(19) Europäisches Patentamt
European Patent Office
Office européen des brevets

(11) Publication number: **0 158 159**
**B1**

(12) # EUROPEAN PATENT SPECIFICATION

(45) Date of publication of patent specification: **05.10.88**

(51) Int. Cl.$^4$: **C 07 C 131/00,** C 07 C 83/00

(21) Application number: **85103052.8**

(22) Date of filing: **16.03.85**

(54) **Process for the synthesis of o-substituted oxime compounds and the conversion thereof into the corresponding hydroxylamine o-substituted.**

(30) Priority: **12.04.84 US 599432**

(43) Date of publication of application:
**16.10.85 Bulletin 85/42**

(45) Publication of the grant of the patent:
**05.10.88 Bulletin 88/40**

(84) Designated Contracting States:
**DE FR GB IT NL**

(58) References cited:
**EP-A-0 023 560**
**EP-A-0 121 701**
**DE-A-2 820 013**

(73) Proprietor: **ALLIED-SIGNAL INC.**
**Columbia Road and Park Avenue P.O. Box 2245R**
**Morristown New Jersey 07960 (US)**

(72) Inventor: **Mathew, Chempolil Thomas**
**c/o Allied Corporation P.O. Box 2245R**
**Morristown NJ 07960 (US)**
Inventor: **Ulmer, Harry Edwards**
**c/o Allied Corporation P.O. Box 2245R**
**Morristown NJ 07960 (US)**

(74) Representative: **Brock, Peter William**
**ALLIED-SIGNAL INC. Law Department**
**48 Leicester Square**
**London WC2H 7LW (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 158 159 B1

**Description**

Background of the invention

1. Field of the invention

The present invention relates to a novel process for the production of O-substituted oximes, which process is especially useful in the production of O-alkyl oximes. More particularly this invention relates to a process for the production of O-substituted oximes under anhydrous conditions.

2. The prior art

The classical method of producing O-alkyl oximes involves reacting an oxime with an organohalide as for example methylbromide or methyl iodide, and an alkali metal alkoxide, such as sodium methoxide. For example, Dunstan and Goulding in J. Chem. Soc., *91*, 628, 1901 have disclosed O-methylation of acetone oxime by reacting acetone oxime with methyl iodide and sodium methoxide.

EPO Patent Application No. 23,560 (*Linhart et al.*, 1980) discloses two procedures for producing O-alkyl oximes. The first procedure involves a modification of the classical method. The modified procedure is a two step process. In the first step of the procedure, an oxime is converted to salt form by reacting an oxime with an alkali metal alkoxide. In the second step of the procedure, the alkali metal salt of the oxime is reacted with an alkyl bromide or alkyl chloride in an aprotic-dipolar solvent. In practice, *Linhart et al.* employed sodium methoxide for conversion of the oxime to the corresponding sodium salt, and then methyl chloride was employed as an alkylating agent. The second procedure disclosed by *Linhart et al.* accomplished O-alkylation of oximes by reacting oximes with powdered sodium hydroxide and an organohalide in the presence of water in an aprotic dipolar solvent.

Similarly, H. S. Anker and H. T. Clarke, "Org. Synth, Col. Vol. III", p. 173 discloses a process for preparation of hydroxylamine O-acetic and by reacting α-bromo-acetic acid and acetoxime in the presence of sodium hydroxide in an aqueous reaction medium and hydrolyzing the resulting acetoxime O-acetic acid. Moreover, Borek and Clarke, J. Am Chem Soc., *58* 2020(1936) describes the use of chloroacetic acid in reaction with acetoxime in which poor yields (46—49%) of a product which was difficult to purify was recorded.

We have unexpectedly discovered a simple process for producing O-substituted oxime compounds, by reacting an Alkali metal or Alkaline Earth metal hydroxide compound with an excess of oxime reactant to form the oxime salt, removing water from the system by azeotropic distillation, and thereafter reacting the oxime salt with an organohalide compound under substantially anhydrous conditions. The process produces O-substituted oximes, especially O-alkyl substituted oximes, in good yield. The economics of the process are excellent in that expensive alkoxides are avoided, and organochlorides instead of bromides and iodides may be employed.

Brief description of the invention

This invention relates to a process for the production of O-substituted oxime compounds of the formula:

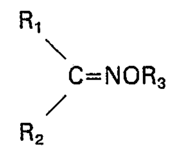

which comprises the steps of:

(a) reacting an oxime compound of the formula:

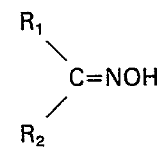

with an Alkali metal or Alkaline Earth metal hydroxide in an excess of the said oxime forming a mixture of the Alkali metal or Alkaline Earth metal salt of said oxime compound, water and said excess of said oxime;

(b) subjecting the reaction mixture of step (a) to azeotropic distillation to remove all or a portion of said water from said mixture;

(c) reacting said Alkali metal or Alkaline Earth metal salt of said oxime compound with a compound of the formula:

$$R_3X$$

wherein:

X is halogen;

$R_1$ and $R_2$ are the same or different and are hydrogen or substituted or unsubstituted aryl, alkyl, cycloalkyl, alkenyl, alkylsulfinyl, arylsulfonyl, arylthio, alkoxyalkyl, alkylthio, alkylsulfonyl, or aralkyl, or $R_1$ and $R_2$ together may form a substituted or unsubstituted alkylene or alkenylene chain completing a cycloalkyl or cycloalkenyl group having from 3 to about 7 carbon atoms within the ring structure, wherein permissible substituents are one or more alkylthio, alkylsulfinyl, alkylaminocarbonyl, dialkylaminocarbonyl, alkylthiocarbonyl, alkyl, alkylsulfonyl, phenoxy, carboxy, amido, alkoxycarbonyl, nitro, alkoxy, arylthio, arylsulfinyl, perfluoroalkyl, cyano or fluorine groups; and

$R_3$ is an organic moiety having a primary, secondary or tertiary carbon atom bonded to the halogen of said organohalide, wherein said carbon atoms of said $R_3$ group may be optionally substituted with one or more substituents moieties that are inert during the process.

Description of the preferred embodiments

The process of this invention consist of two essential steps. A preferred embodiment of the first essential step of the process of this invention is described schematically in the following Reaction Step A:

Reaction Step A

$$R_1R_2C{=}N{-}OH + MOH \rightarrow R_1R_2{-}C{=}NOM + H_2O$$
$$\text{(I)} \qquad\qquad\qquad \text{(II)}$$

wherein $R_1$ and $R_2$ are as defined hereinabove, and M is an Alkali or Alkaline Earth metal. The first essential step of the process of this invention to form the corresponding Alkali metal or Alkaline Earth metal salt of the oxime compound is conveniently performed by reacting an Alkali metal or Alkaline Earth metal hydroxide, and an excess of the oxime compound of the formula $R_1R_2C{=}NOH$.

In those instances where the oxime is a liquid under the reaction conditions, the oxime can be employed as the reaction solvent, or a combination of the oxime and a suitable organic co-solvent can comprise the reaction solvent. In those instances where the oxime is a solid under the reaction mixture a co-solvent is used, and the oxime/co-solvent solution functions as the reaction solvent. As used herein, a "suitable organic solvent" is any organic solvent which does not react with the hydroxide and oxime reactants under the reaction conditions of the process, which is capable of forming a solution ,of the oxime and hydroxide reactants and which aids in the formation of azeotropic mixtures when distilled with water. In the preferred embodiments of the invention, the reaction is carried out with a co-solvent. Illustrative of useful co-solvents are non-polar solvents as for example aliphatic and cycloaliphatic hydrocarbons, such as hexane, cyclohexane, heptane, cyclopentane, pentane, isooctane, and the like; aromatic solvents such as benzene, toluene, xylene and the like; and halohydrocarbons such as carbon tetrachloride, methylene dichloride, chlorofluoromethane, dichlorodifluoroethane, trichlorotrifluoroethane, chloroform, and the like. Preferred non-polar organic solvents for use in the practice of this invention are fluorohydrocarbon solvents, hydrocarbon solvents and aromatic solvents, and particularly preferred for use in the process are aromatic solvents such as toluene and xylene, and hydrocarbon solvents such as pentane, isooctane, cyclohexane and the like.

The amount of solvent is not critical and can vary widely. Usually, the amount of solvent will vary from about 5 to about 200 percent by weight based on the total weight of the oxime reactant. The preferred amount of solvent is from about 50 to about 100 weight percent by weight of the oxime reactant. Greater amounts of solvent can of course be used, except such amounts merely dilute the components of the reaction mass with no particular advantage being obtained.

Oxime compounds which are useful as reactants in the conduct of the process of this invention are of the formula:

$$R_1R_2C{=}NOH$$

in which $R_1$ and $R_2$ are as described above. Such compounds are well known to those of skill in the art and include such compounds as acetaldehyde oxime, propionaldehyde oxime, n-butyraldehyde oxime, isobutyraldehyde oxime, n-valeraldehyde oxime, pivalaldehyde oxime, acetone oxime, methylethyl ketone oxime, 2-pentanone oxime, 3-pentanone oxime, 2-hexanone oxime, ethylisobutyl ketone oxime, vanillin oxime, phenylacetaldehyde oxime, methylisobutyl ketone oxime, benzaldehyde oxime, acetophenone oxime, propiophenone oxime, n-butyrophenone oxime, cyclohexanecarboxaldehyde oxime, cyclopentanecarboxaldehyde oxime, 2,2-dimethylcyclohexanecarboxaldehyde oxime, cycloheptanecarboxaldehyde oxime, 2-methoxyacetaldehyde oxime, 2-ethoxypropionaldehyde, 3-butoxybutyraldehyde oxime, benzylethylketone oxime, cyclohexylmethyl ketone oxime, 2-propoxyvaleraldehyde oxime, and the like. Other useful oxime reactants include 2-phenylpropionaldehyde oxime, 3-phenylvaleraldehyde oxime, benzophenone oxime, cyclohexanone oxime, cyclopentanone oxime, o-tolualdehyde oxime, m-tolualdehyde oxime, 2-benzyl propionaldehyde oxime, 2-ethyl-2-phenylacetaldehyde oxime, and the like. As was noted above, $R_1$ and $R_2$ substituents of the aforementioned compounds can be substituted with one or more functional groups which are relatively non-reactive under the reaction conditions employed in the process. Illustrative of such non-reactive functional groups are fluorine, alkoxy nitro, cyano, alkylthio, arylsulfinyl, arylsulfonyl, alkyl, arylthio, alkylsulfinyl, alkylsulfonyl, phenoxy, carboxy, amido, alkoxycarbonyl, perhaloalkyl, and like non-reactive functional groups.

Preferred for use in the practice of this invention are oxime compounds of the above formula in which $R_1$ and $R_2$ are the same or different and are hydrogen, or substituted or unsubstituted phenyl, alkylphenyl having from 7 to about 14 carbon atoms, alkyl having from 1 to about 7 carbon atoms and phenylalkyl having from 7 to about 14 carbon atoms. Particularly preferred for use in the process of this invention are oxime compounds in which $R_1$ and $R_2$ are hydrogen, or substituted or unsubstituted alkyl having from 1 to about 4 carbon atoms, alkylphenyl having from 7 to about 11 carbon atoms, or phenylalkyl having from 7 to about 11 carbon atoms wherein permissible substituents are one or more alkoxycarbonyl, carboxy, alkylthio, nitro, cyano, and trifluoromethyl. Amongst these particularly preferred embodiments, most preferred are those embodiments in which $R_1$ and $R_2$ are hydrogen, or alkyl having from about 1 to about 4 carbon atoms unsubstituted or substituted with one or more alkoxycarbonyl or carboxy substituents, with those of the aforementioned particularly preferred compounds in which $R_1$ and/or $R_2$ is unsubstituted or substituted methyl or ethyl being especially preferred.

The oxime compounds utilized as reactants in the process of this invention can be conveniently prepared according to conventional methods. For example, these compounds can be conveniently

prepared by reacting an appropriate aldehyde or ketone with hydroxylamine salts, optionally in the presence of an Alkali metal hydroxide, an Alkali metal carbonate or ammonia. Another method involves reacting the corresponding aldehyde or ketone in a water medium with sodium nitrite, sodium bisulfite and sulfur dioxide.

Any alkali metal or Alkaline Earth metal hydroxide compound can be employed in the first step of the process of this invention such as lithium hydroxide, potassium hydroxide, calcium hydroxide, magnesium hydroxide, and sodium hydroxide. Alkali metal hydroxide compounds are preferred for use in the practice of this invention. Particularly preferred for use are potassium hydroxide and sodium hydroxide, and most preferred for use in the practice of this invention is sodium hydroxide. The form of the Alkali metal or Alkaline Earth metal hydroxide is not critical. The hydroxide can be added in solid form, or can be added in solution form, as for example dissolved in the oxime reactant or in water.

The reaction temperature of the first step of the process of this invention can vary from the freezing point of the reaction mixture up to the temperature at which oxime salt reaction product becomes susceptible to decomposition. In the preferred embodiments of the invention, reaction temperatures will vary from about 0°C to about 150°C, and in the particularly preferred embodiments of this invention the reaction temperatures will vary from about 25°C to about 120°C. Amongst these particularly preferred embodiments, most preferred are these embodiments in which the reaction temperature varies from about 50°C to about 100°C.

After completion of the first step of the process of this invention, the reaction mixture is subjected to azeotropic distillation employing conventional procedures to remove all or a portion of the water added to the reaction mixture or formed during the reaction of the first step. Such procedures are well known to those of skill in the art and will not be described herein in any great detail. In some instance, the excess of the oxime and the water by-product may form an azeotropic mixture when distilled. In other instances, one of the above referenced co-solvents can aid in the formation of the azeotrope is added. In the preferred embodiments of this invention the azeotropic distillation is continued until the reaction mixture contains less than about 200 ppm of water, and in the particularly preferred embodiments is continued until the reaction mixture contains less than about 1000 ppm water. Amongst these particularly preferred embodiments of the invention, most preferred are those embodiments in which the azeotropic distillation is continued until the mixture contains less than about 100 ppm water.

The Alkali metal or Alkaline earth metal salt of the oxime compound can be reacted *in situ* with an appropriate organo halide compound in the second essential step of the process of this invention, or can be isolated from the reaction mixture and purified for use in the second step of the process at some later time using conventional techniques known to those of skill in the art. In the preferred embodiments of the invention the salt and the organo halide compound are reacted *in situ.*

The second essential step of the process of this invention is described schematically in the following Reaction Step B:

Reaction Step B

$$R_1R_2C{=}NOM+R_3X \rightarrow R_1R_2C{=}NOR_3+MX$$

wherein $R_1$, $R_2$ M and X are as described above. In step 2 of the process of this invention, the reaction of the Alkali metal or Alkaline Earth metal salt of the oxime compound, and the organo halide compound is carried out in an organic solvent which is non-reactive with the salt of the oxime and the organo halide reactant. In the preferred embodiments of the invention, this solvent is selected from the group consisting of solvents which are permissible for use in the first step of the process of this invention. Generally, essentially about stoichiometric quantities of the organo halide and oxime salt reactants are employed, although, it should be understood that the quantity of either reactant can vary from about stoichiometric to as much as a 50 percent excess or even more. In the preferred embodiments of this invention, the quantities of the reactants employed will vary from about stoichiometric to an excess of 10 percent.

Useful organo halide compounds for use in the process of this invention include compounds in which $R_3$ of the above formula is alkyl such as methyl, ethyl, propyl, hexyl, isopropyl, isobutyl, decyl, pentyl and the like; in which $R_3$ is alkenyl such as allyl, 2-pentenyl, 3-butenyl, 3-pentenyl, 4-hexenyl, and the like; and in which $R_3$ is alkynyl as for example propargyl, 2-pentynyl, 3-hexynyl, 2-butynyl, 3-decynyl, 2-hexynyl, 4-hexynyl, 4-octynyl, and the like. Other useful organo halide reactants are those in which $R_3$ of the above formula is a cyclic group as for example a cycloalkyl group such as cycloheptyl, cyclohexyl, cyclobutyl, cyclopentyl, and the like, or a cycloalkenyl group such as 2-cyclohexenyl, 3-cycloheptenyl, 2-cyclopentenyl and the like. Illustrative of still other useful organo halide reactants for use in the process of this invention are those in which $R_3$ is an aromatic function, such as benzene, 2-methylbenzene, benzyl, phenethyl and the like; alkylthiocarbonyl such as methylthiocarbonyl, ethythiocarbonyl and the like; alkoxycarbonyl, such as methoxycarbonyl, ethoxycarbonyl, propoxycarbonyl and the like, aryl and aryloxycarbonyl such as phenylcarbonyl, phenoxycarbonyl, 2,4-dichloro-6-methoxyphenylcarbonyl, 2,4-dichloro-6-methoxyphenoxycarbonyl and the like; heterocarbonyl, such as 1-thienylcarbonyl, 1-furanylcarbonyl and the like; alkylaminocarbonyl and dialkylaminocarbonyl, such as methylaminocarbonyl, dimethylaminocarbonyl and the like; alkylsulfonyl, such as methylsulfonyl, ethylsulfonyl and the like; alkyl aminosulfonyl such as

methylaminosulfonyl, dimethylaminosulfonyl and the like; and aliphatic, araliphatic, heterocyclic or cycloaliphatic acyl groups such as acetyl, propionyl, 2-thienylcarbonyl, hexanoyl, butanoyl, pentanoyl, octanoyl, isopropanoyl, 2-furanylcarbonyl, 2-pyranylcarbonyl, 4-pyridylcarbonyl, 3-cyclohexylcarbonyl, phenethylcarbonyl, and the like. Of course, as previously noted these $R_3$ functions may be unsubstituted or substituted with one or more functional groups which are non-reactive under process conditions of the second essential step of the process of this invention. Such permissible functional groups include perfluoroalkyl, alkyl, aryl, alkoxy, cyano, nitro, amido, arylthio, alkylsulfinyl, alkylsulfonyl, aminocarbonyl, carboxy, alkylthio, alkoxycarbonyl, alkoxyalkyl, and the like. Preferred substituents are amido, carboxy and alkoxycarbonyl, preferably substituted to an alkyl group having from 1 to about 7 carbon atoms.

While any organo halide compound of the formula $R_3X$ can be used in the process of this invention, organo chloride compounds are used in the preferred embodiments of the invention because of greater availability and lower cost. In the particularly preferred embodiments of this invention, organo chloride compounds of the formula $R_3Cl$ in which $R_3$ is substituted or unsubstituted alkyl having from 1 to about 7 carbon atoms, phenylalkyl compound having from 7 to about 14 carbon atoms, and alkenyl having from 2 to 7 carbon atoms wherein permissible substituents are one or more alkoxycarbonyl, carboxy, alkylthio, amido, aminocarbonyl, alkylthiocarbonyl, nitro, cyano, or trifluoromethyl groups are used. Amongst the organo chlorides for use in the particularly preferred embodiments of the invention, most preferred are those organo chlorides in which $R_3$ is alkyl having from 1 to about 5 carbon atoms, alkenyl having from 2 to about 5 carbon atoms, and phenylalkyl having from 1 to about 11 carbon atoms either unsubstituted or substituted with one or more alkoxycarbonyl, amido or carboxy substituents.

Organo halide compounds utilized as reactants in the process of this invention as well as methods for their preparation are well known in the art. For example, such compounds can be readily prepared by reacting with halogen, as for example chlorine, with an appropriate organo compound.

The temperature employed in the organo halide addition step are usually in the same range as those employed in the first step of the invention. Temperatures within the range of from about 0°C to about 100°C are preferred, and reaction temperatures of from about 25°C to about 80°C are particularly preferred.

The process of this invention is carried out over a period of time sufficient to produce the desired compound in adequate yield. Reaction times are influenced to a significant degree by the reactants; the reaction temperature; the concentration and choice of reactants; the choice and concentration of reaction solvent and by other factors known to those skilled in the art. In general, residence times can vary from about a few minutes to 24 hours or longer. In most instances, when employing preferred reaction conditions, reaction times will be found to vary from about 1 hour to about 3 hours.

The process of this invention can be conducted in a batch, semicontinuous or continuous fashion. The reactants and reagents may be initially introduced into the reaction zone batchwise or they may be continuously or intermittently introduced in such zone during the course of the process. Means to introduce and/or adjust the quantity of reactants introduced, either intermittently or continuously into the reaction zone during the course of the reaction can be conveniently utilized in the process especially to maintain the desired molar ratio of the reaction solvent, reactants and reagents. The reaction can be conducted in a single reaction zone or in a plurality of reaction zones, in series or in parallel or it may be conducted intermittently or continuously in an elongated tubular zone or series of such zones. The materials of construction employed should be inert to the reactants during the reaction and the fabrication of the equipment should be able to withstand the reaction temperatures and pressure.

The reaction zone can be fitted with one or more internal and/or external heat exchanger(s) in order to control undue temperature fluctuations, or to prevent any possible "runaway" reaction temperatures. In preferred embodiments of the process, agitation means to vary the degree of mixing the reactions mixture can be employed. Mixing by vibration, shaking, stirring, rotation, oscillation, ultrasonic vibration or the like are all illustrative of the type of agitation means contemplated. Such means are available and well known to those skilled in the art.

The product O-substituted oxime compound can be isolated from the reaction mixture and purified employing conventional techniques. Illustrative of such techniques are evaporation, distillation, solvent extraction and recrystallization. More specifically, isolation of the O-substituted oxime compound can be accomplished by filtering off the halide salt and subjecting the filtrate to azeotropic distillation. The pot residue can be extracted with a suitable solvent and the extract concentrated under reduced pressure to provide the desired O-substituted oxime compound.

The O-substituted oximes that may be produced by the process of this invention are valuable as intermediates in the production of O-substituted hydroxylamines, as for example, O-methyl hydroxylamine, O-ethyl hydroxylamine, O-benzyl hydroxylamine, O-allyl hydroxylamine, O-carboxymethylhydroxylamine, O-ethoxy-carbonylmethylhydroxylamine, and the like, which are themselves valuable as pharmaceutical and agricultural products and intermediates. O-Substituted hydroxylamines may be produced by hydrolysis (preferably acid hydrolysis using non-

oxidizing acids such as hydrochloric acid, sulfuric acid, phosphoric acid, p-toluene sulfonic acid, and the like) of the O-substituted oximes produced by this invention, wherein the hydrolysis is conducted for a period of time between about a few minutes and about 25 hours at a temperature between ambient and about 150°C with about 40°C to about 120°C generally being preferred. In some cases, the hydrolysis may be conducted in the oxime product solution without isolation of the O-substituted oxime. For example, O-methyl hydroxylamine, O-carboxymethyl hydroxylamine, O-ethoxycarbonylmethyl hydroxylamine, O-allyl hydroxylamine, and O-benzyl hydroxylamine may be produced by hydrolysis of the corresponding O-substituted oxime in the product solution. However, best results are generally obtained by isolating the O-substituted oxime and then hydrolyzing said oxime. Hydrolysis of O-allyl 2-butanone oxime, O-methyl benzaldehyde oxime, O-carboxymethyl 2-butanone oxime and ethoxycarbonyl methyl 2-propanone oxime produced in accordance with the process of this invention produces the corresponding O-substituted hydroxylamine in good yield.

In a preferred embodiment of this invention, O-methyl benzaldehyde oxime, and the O-carboxymethyl and O-ethoxycarbonylmethyl derivatives of methyl ethyl ketone oxime or dimethyl ketone oxime are produced. These preferred O-substituted oxime compounds can also be employed as intermediates in the production of N,O-substituted hydroxylamine, such as N,O-dimethyl hydroxylamine a valuable intermediate in the production of pesticides. N,O-substituted hydroxylamine may be produced by reacting the O-substituted oxime compound produced in accordance with this invention with dimethyl sulfate to produce a sulfate salt intermediate; without isolation of the sulfate salt intermediate, the intermediate may then be converted *via* hydrolysis, preferably acid hydrolysis, to produce a product mixture comprising N,O-substituted hydroxylamine, such as N,O-dimethyl hydroxylamine and the aldehyde or ketone. N,O-substituted hydroxylamine, such as N,O-dimethyl hydroxylamine, may be separated from the product mixture using conventional procedures.

The following examples are presented to more particularly illustrate the process of this invention, but in no way are the examples to be construed as limitations upon the process.

Example 1

Methyl ethyl ketoxime (100 g; excess) was mixed with a 50% solution of sodium hydroxide (40 g; 0.50 mol) and toluene (100 mL) in a 3-necked 500 mL flask fitted with a thermometer, reflux condenser and a Dean-Stark water separator. The mixture was stirred using a magnetic stirring bar and heated under reflux over a heating mantle. After about one hour, when no more water (28 g) collected in the Dean-Stark apparatus a clear solution resulted in the flask. It was cooled down to about 50°C and to this was added a solution of monochloroacetic acid (18.9 g; 0.2 mol) in toluene (100 mL) slowly with stirring. On completion of addition, the mixture, which gradually turned cloudy was heated at about 80°C for 3 hours.

The yellowish slurry was then cooled and diluted with water (500 mL) and pH adjusted to 7 (from pH 12.3) using conc. HCl (10 g), and the liquid-liquid mixture placed in a 1 liter 3-necked flask fitted with thermometer, Clasisen head and condenser. A colorless azeotropic distillate was collected (640 mL of two-phase mixture) till the pot temperature reached and remained at 100—100.5°C. Most of the distillate came over at a pot temperature of 87—88°C. Completion of removal of all distillable organics was checked by gas chromatographic analysis of a drop of the distillate from the tip of the condenser (no MEK oxime or toluene detected).

The yellowish brown, aqueous pot residue was cooled in ice bath (0—5°C) and slowly acidified with concentrated HCl (25 g). pH of the solution was 0.6. The cold solution was then saturated with sodium chloride and extracted with toluene (8×100 mL) and the clear and virtually colorless toluene extract was concentrated under reduced pressure. The light yellowish oily liquid (28.5 g) that resulted was analyzed by gas chromatography to be 83.5% pure methyl ethyl ketoxime O-acetic acid, the other two major components being toluene and methyl ethyl ketoxime. Yield 82.1% based on chloroacetic acid used.

Distillation of the liquid furnished a colorless oily liquid (B.P. 92—95°C @ 0.9 mm Hg), the identity of which was confirmed by $^1H$ and $^{13}C$ NMR analyses to be methyl ethyl ketoxime O-acetic acid.

Example 2

A 500 ML, 3-neck flask was fitted with Dean-Stark water separator fitted with a condenser dropping funnel, a thermometer and a magnetic stirring bar was placed in it. Nitrogen blanket was provided and methyl ethyl ketoxime (100 g; 1.15 mol) mixed with hexane (75 ML) was added to the flask. Sodium hydroxide flakes (4.5 g; 0.11 mol) was added, and the mixture was heated under reflux with stirring over a stir-plate. Within 1 hour a clear solution resulted and no more water was found collecting in the Dean-Stark separator. A total of 2.5 ML of water was removed.

After cooling to 10°C, and removing the water separator from the system, 1-chloropropane (7.9 g; 0.1 mol) were added slowly with stirring from the dropping funnel. No exotherm was noticed. The mixture was then heated under reflux for a total of 40 hours over 5 days, by which time a white precipitate of sodium chloride collected. The reaction mixture was cooled and filtered, and the clear filtrate of hexane solution was washed repeatedly (3×, 25 ML) with a 20% aqueous sodium hydroxide solution to remove excess methyl ethyl ketoxime. The colorless hexane solution was analyzed by gas chromatography and found to contain methyl ethyl ketoxime-O-propyl ether (9.2 g). Yield was 71.3%.

Example 3

Into a 500 ML 3-neck flask set up as in Example 2, was charged a solutional cyclohexanone oxime (75 g; 0.66 mol) in toluene (150 ML). Sodium hydroxide pellets (4.5 g; 0.11 mol) were added, and the mixture was heated under reflux with stirring over a stir-plate until no more water collected in the water separator. The solution was then cooled to ambient temperature and benzyl chloride (12.7 g; 0.1 mol) was added slowly with stirring. No exotherm was noticed. The clear mixture was then heated at 90°C, and within half-hour turned turbid. After heating for a total of 2 hours at 90°C with stirring, the white slurry was cooled and mixed with water (50 ML). The top organic phase was separated and washed with 20% aqueous sodium hydroxide solution (3×, 35 ML). The clear toluene solution was analyzed by gas chromatography and found to contain cyclohexanone oxime-O-benzyl ether (15.5 g). Yield was 71.3%.

Example 4

Into a 500 ML 3-neck flask set up as in Example 2 was charged a solution of methyl ethyl ketoxime (100 g; 1.15 mol) in hexane (100 ML). To the solution was added sodium hydroxide (5 g; 0.13 mol), and the mixture heated under reflux until no more water collected in the water separator.

The clear solution was placed in a 500 ML stainless steel autoclave and cooled in dry ice bath. In the meantime ethyl chloride (6.5 g; 0.1 mol) was collected in a dry ice-cooled trap from a lecture bottle. The ethyl chloride was quickly added to the autoclave, and the equipment was sealed. Agitation was started and the autoclave was slowly heated to 90°C and maintained at that temperature for a total of 5 hours. After cooling the contents of the autoclave (a white slurry) were transferred using water (50 ML). The top hexane phase was collected and washed with 15% aqueous NaOH (3×, 35 ML), and the washings were washed with the aqueous bottom phase above. The clear and colorless hexane solution was analyzed by gas chromatography and found to contain methyl ethyl ketoxime-O-ethyl ether (8.8 g). Yield was 76.5%.

The aqueous phase and the aqueous NaOH washings were neutralized using hydrochloric acid, with cooling, and extracted with hexane to recover all of the unused methyl ethyl ketoxime.

Example 5

A mixture of acetaldehyde oxime (10 g; 0.17 mol) and hexane (150 ML) was placed in a 250 ML 3-neck flask fitted with thermometer, Dean-Stark water separator and are flux water condenser. A nitrogen blanket was provided, and sodium hydroxide pellets (3 g; 0.075 mol) were added. The mixture was stirred and heated under reflux until water ceased to collect in the Dean-Stark separator. The resulting clear and colorless solution was cooled, and benzyl chloride (7 g; 0.055 mol) was added slowly with agitation. The solution was heated at reflux (70°C) for 6 hours, and a thin white slurry was formed. The solution was stirred with water (50 ML) and the top hexane phase collected. The hexane solution was then washed three times with 15% sodium hydroxide solution (35 ML each) to remove excess acetaldehyde oxime. The resulting hexane solution was analyzed by gas chromatography and found to contain acetaldehyde oxime-O-benzyl ether (5.8 g). Yield was 70.7%.

The aqueous phase and the aqueous sodium hydroxide washings were treated with concentrated HCl to pH 7, and then extracted with hexane to recover all unused acetaldehyde oxime.

Example 6

In a 500 ML 3-neck flask provided with nitrogen blanket and fitted with a thermometer, a Dean-Stark water separator and reflux condenser was placed a mixture of methyl ethyl ketoxime (100 g; 1.15 mol) and toluene (100 ML). Sodium hydroxide pellets (9 g; 0.225 mol) were added and the mixture was stirred using a magnetic stirring bar while heating at reflux. A total of 4.5 g of water were collected. After water ceased to collect, the resulting clear solution was cooled to 24°C and 3-chloropropene (15.3 g; 0.2 mol) was slowly added from a dropping funnel with stirring. The resulting solution was heated at reflux (115°C) for 4 hours. On cooling, the reaction mixture was washed with an aqueous 20% solution of sodium hydroxide (3×, 35 ML) to remove unreacted methyl ethyl ketoxime. The washed toluene solution was analyzed by gas chromatography to ensure that all unreacted methyl ethyl ketoxime was removed. On evaporation of toluene, a light yellow oily liquid was collected (18.9 g), which was identified by Mass Spectrometry as methyl ethyl ketoxime-O-propenyl ether. Yield was 74.4%.

Unused methyl ethyl ketoxime was recovered from the aqueous phase mixed with the aqueous NaOH washings by neutralization with concentrated HCl to pH 7 and extraction with toluene.

Example 7

A mixture of acetone oxime (75 g; 1.03 mol) and hexane (150 ML) was placed in a 500 mL 3-neck flask fitted with a thermometer, a Dean-Stark apparatus, a reflux condenser and a dropping funnel. A magnetic stirring bar was placed in the flask and nitrogen atmosphere was provided. To the mixture was added with stirring over a stir-plate sodium hydroxide (4.5 g; 0.11 mol), and the mixture heated under reflux until no more water collected in the Dean-Stark apparatus. The resulting dry clear solution was cooled to 20°C, and 3-chloropropene (7.7 g; 0.1 mol) was added with stirring. The solution was heated at reflux (71°C) for 8 hours and then cooled to ambient temperature. After washing the mixture with a 20% aqueous solution of sodium hydroxide (3×, 35 ML), the hexane phase was collected and was concentrated. the concentrated hexane phase was analyzed by gas chromatography and found to contain acetone oxime-O-propenyl ether (8.2 g). Yield was 72.6%.

Example 8

A 2-liter 3-neck flask fitted with a thermometer, Dean-Stark water separator and reflux condenser was provided with nitrogen blanket. A mixture of acetone oxime (625 g; 8.56 moles) and toluene (600 mL) was placed in the flask. To the mixture was added sodium hydroxide pellets (132 g; 3.3 moles), and the contents heated under reflux with stirring using an over-head stirrer. A total of 60 g of water was removed. When no more water distilled over, the contents were cooled to 25°C and a solution of chloroacetic acid (141.8 g; 1.5 moles) was added slowly from a dropping funnel. The exothermic reaction was controlled so that the temperature did not exceed 70°C. After addition, the resulting slurry was stirred for 1 hour at 60—70°C.

On cooling the slurry was mixed with ice and water, and concentrated hydrochloric was added to bring pH to 10. The top organic phase was separated from the bottom aqueous phase and the aqueous phase extracted with toluene (6× 100 ml) to remove all acetone oxime. The clear aqueous solution was then cooled to 0°C in an ice bath and more hydrochloric acid (155 g) added to lower pH to 1. Toluene extraction (6×100 ml) was carried out quickly keeping the aqueous solution cold throughout. The virtually colorless combined toluene extracts were stripped of the solvent on a rotovap, and acetone oxime-O-acetic acid was collected as a white crystalline solid (149.9 g), M.P. 75—76°C. Yield was 76.3%. The identity of the product was confirmed by $^{13}C$ and proton NMR analyses.

A portion of the above crystalline solid (100 g) was mixed with (225 g) concentrated hydrochloric acid, distilled water (600 mL) and a small amount of (20 mg) of hydroquinone. The clear solution was set up for distillation with a Claisen head and condenser under reduced pressure (~60 mm Hg) with a nitrogen bleed into the system. The pot temperature was held below 50°C throughout and the distillation continued until no more acetone stripped over (387 g total distillate). The clear aqueous pot residue was analyzed by $^{13}C$-NMR and found to contain 14.2% of hydroxylamine-O-acetic acid hemihydrochloride. Yield was 91.4%.

Example 9

A mixture of methyl ethyl ketoxime (100 g; 1.15 mol) and toluene (150 ml) was placed in a 3-neck 500 mL flask. The flask was fitted with a Dean-Stark apparatus, a reflux condenser and a thermometer. A nitrogen atmosphere was provided, and sodium hydroxide pellets (4.5 g; 0.11 mol) were added. The resulting mixture heated under reflux with stirring using a magnetic stirrer, and 2.5 ml water was collected. The mixture appeared dry and was cooled to ambient temperature.

The above solution was placed in a 500 mL autoclave and cooled in a dry ice bath. Methyl chloride (6 g; 0.12 mol) collected as a liquid from a cylinder was quickly added to the autoclave and sealed. The autoclave was then slowly warmed with agitation, heated to 90°C and maintained at that temperature for 5 hours. On cooling a thin slurry was collected from the autoclave, which was mixed with 35 mL of an aqueous 20% solution of sodium hydroxide. The top organic phase was collected and was extracted twice with similar portions of the sodium hydroxide solution, and distilled on a rotovap to remove toluene. The resulting colorless liquid (7.8 g) was characterized as methyl ethyl ketoxime-O-methyl ether by Mass-Spectrometry.

Example 10

Following the procedure of Example 1, methyl ethyl ketoxime in toluene was reacted with sodium hydroxide in a 3-necked 500 mL flask fitted with a thermometer, reflux condensor and a Dean-Stark water separator until water ceased to be collected. The resulting mixture was then reacted with chloroacetic acid. At the end of the reaction of chloroacetic acid, the resulting yellowish slurry was cooled, and diluted with water (500 mL). The pH was adjusted to 10 by addition of hydrochloric acid. The top organic phase which consisted primarily of toluene and excess Methylethyl Ketoxime was collected. The aqueous bottom phase was extracted repeatedly (6×, 100 ML) with toluene, and the organic extracts mixed with the major organic fraction.

The pH of the washed aqueous solution was adjusted to 0.8 by addition of concentrated HCl, with careful cooling (0—5°C) and stirring. The cold solution was saturated with sodium chloride, and quickly extracted with fresh toluene (6×, 100 ML). The toluene extracts were combined, and then evaporated on a rotovap under reduced pressure to provide a light yellow oil containing 94.4% methyl ethyl ketoxime-O-acetic acid (20.2 g). Yield was 85.3%.

The methyl ethyl ketoxime-O-acetic acid was mixed with concentrated HCl (50 g), distilled water (150 ML) and hydroquinone (10 mg) and the mixture was distilled under reduced pressure (50—60 mm Hg) at a pot temperature of 40—45°C with a nitrogen atmosphere. After 2 hours no more methyl ethyl ketone was detected in the distillate (gas chromatography). The residual solution was cooled and recovered (85.4 g). Analysis of the aqueous solution by $^{13}C$-NMR showed the presence of 19.8% of hydroxylamine-O-acetic acid hemihydrochloride. Yield was 90.9%.

**Claims**

1. A process for the preparation of O-substituted oxime compounds of the formula:

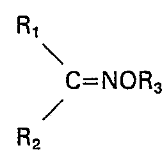

which comprises the steps of:

(a) reacting an oxime compound of the formula:

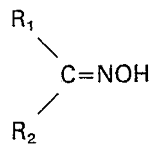

with an Alkali metal or Alkaline Earth metal hydroxide compound in an excess of the said oxime compound to form a mixture of the Alkali metal or Alkaline Earth metal salt of said oxime compound, water and said excess of said oxime;

(b) subjecting the reaction mixture of step (a) to azeotropic distillation to remove all or a portion of said water from said mixture; and

(c) reacting said Alkali metal or Alkaline Earth metal salt of said oxime compound with a compound of the formula:

$$R_3X$$

wherein:

X is halogen;

$R_1$ and $R_2$ are the same or different and are hydrogen, or substituted or unsubstituted arylthio, arylsulfonyl, arylsulfinyl, alkylsulfinyl, alkylsulfonyl, alkyl, alkynyl, cycloalkyl, alkenyl, alkoxy, alkoxyalkyl, alkylthio, aryl or aralkyl, or $R_1$ and $R_2$ together may form a substituted or unsubstituted alkylene or alkenylene chain completing a cycloalkyl or cycloalkenyl group containing from 3 to about 7 carbon atoms within the ring structure, wherein permissible substituents are one or more alkyl, alkoxy, alkylthio, amido, carboxy, fluoro, nitro, cyano, alkoxycarbonyl, perfluoroalkyl, arylthio, phenoxy, alkylsulfinyl, alkylsulfonyl, arylsulfinyl, alkylaminocarbonyl, dialkylaminocarbonyl, alkylthiocarbonyl, or arylsulfonyl groups; and

$R_3$ is an organic moiety having a primary, secondary or tertiary carbon atom bonded to the halogen of said organohalide wherein said carbon atoms of said $R_3$ group may be optionally substituted with one or more substituents that are inert during the process.

2. A process according to claim 1 wherein said reaction mixture subjected to azeotropic distillation in step (b) includes one or more non-polar non-reactive organic solvents which form azeotropic mixtures when distilled with water and said oxime compound.

3. A process according to claim 2 said reaction of step (a) is carried out in the presence of one or more non-reactive, non-polar organic solvents which form azeotropic mixtures when distilled with water and said oxime compound.

4. A process in accordance with claim 3 wherein said solvent is selected from the group consisting of aliphatic and cycloaliphatic hydrocarbon solvents, aromatic solvents, and halohydrocarbon solvents.

5. A process according to claim 1 wherein $R_3$ is alkenyl, alkyl or alkyl substituted with one or more carboxy, amido, cyano or alkoxycarbonyl groups.

6. A process according to claim 1 wherein said oxime compound is reacted with an Alkali metal hydroxide to form the corresponding Alkali metal salt of said oxime compound.

7. A process in accordance with claim 1 wherein said process is conducted at a temperature between 20°C and 150°C.

8. A process in accordance with claim 1 wherein $R_1$ and $R_2$ are the same or different and are hydrogen, alkyl, phenyl or phenylalkyl.

9. The process in accordance with claim 1 which further comprises hydrolyzing said O-substituted oxime of the formula:

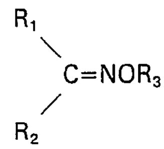

to form an O-substituted hydroxylamine of the formula:

$$H_2N{-}OR_3$$

10. The process of claim 1 wherein said oxime is reacted with dimethyl sulfate to produce a sulfate salt intermediate, which said sulfate salt intermediate then being hydrolyzed to produce the corresponding N,O-substituted hydroxylamine, and an aldehyde or ketone compound.

**Patentansprüche**

1. Verfahren zur Herstellung O-substituierter Oximverbindungen der Formel

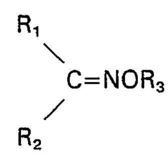

dadurch gekennzeichnet, daß man

(a) ein Oxim der Formel

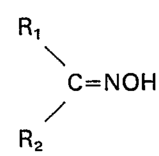

mit einem Alkali- oder Erdalkalihydroxid im Überschuß zu der Oximverbindung unter Bildung eines Gemisches des Alkali- oder Erdalkalisalzes der Oximverbindung, Wasser und dem Überschuß an dem Oxim umsetzt;

(b) das Reaktionsgemisch der Stufe (a) einer azeotropischen Destillation unterwirft, um das gesamte Wasser oder einen Teil des Wassers von dem Gemisch abzutrennen; und

(c) das Alkali- oder Erdalkalisalz des Oxims mit einer Verbindung der Formel

$$R_3X$$

umsetzt, worin

X Halogen ist,

$R_1$ und $R_2$, die gleich oder verschieden sind, Wasserstoff oder substituiertes oder unsubstituiertes Arylthio, Arylsulfonyl, Arylsulfinyl, Alkylsulfinyl, Alkylsulfonyl, Alkyl, Alkynyl, Cycloalkyl, Alkenyl, Alkoxy, Alkoxyalkyl, Alkylthio, Aryl oder Aralkyl bedeuten, oder $R_1$ und $R_2$ zusammen eine substituierte oder unsubstituierte Alkylen- oder Alkenylenkette, die eine Cycloalkyl- oder Cycloalkenylgruppe mit 3 bis etwa 7 Kohlenstoffatomen in der Ringstruktur vervollständigt, bilden, wobei zulässige Substituenten ein oder mehrere Alkyl-, Alkoxy-, Alkylthio-, Amido-, Carboxy-, Fluoro-, Nitro-, Cyano-, Alkoxycarbonyl-, Perfluoralkyl-, Arylthio-, Phenoxy-, Alkylsulfinyl-, Alkylsulfonyl-, Arylsulfinyl-, Alkylaminocarbonyl-, Dialkylaminocarbonyl-, Alkylthiocarbonyl- oder Arylsulfonyl-gruppen sind; und $R_3$ ein organischer Anteil mit einem an das Halogen des Organohalogenids gebundenen sekundären oder tertiären Kohlenstoffatom ist, worin die Kohlenstoffatome der Gruppe $R_3$ gegebenenfalls mit einem oder mehreren Substituenten, die während des Verfahrens inert sind, substituiert sein können.

2. Verfahren nach Anspruch 1, dadurch gekennzeichnet, daß das der azeotropischen Destillation in Stufe (b) unterworfene Reaktionsgemisch ein oder mehrere nicht-polare nicht-reaktive organische Lösungsmittel enthält, die azeotropische Gemische bilden, wenn sie mit Wasser und der Oximverbindung destilliert werden.

3. Verfahren nach Anspruch 2, dadurch gekennzeichnet, daß die Reaktion der Stufe (a) in Gegenwart von einem oder mehreren nichtreaktiven nicht-polaren organischen Lösungsmitteln, die, wenn sie mit Wasser und der Oximverbindung destilliert werden, azeotropische Gemische bilden, durchgeführt wird.

4. Verfahren nach Anspruch 3, dadurch gekennzeichnet, daß das Lösungsmittel aus der Gruppe der aliphatischen und cycloaliphatischen Kohlenwasserstofflösungsmittel, aromatischen Lösungsmittel und Halogenkohlenwasserstofflösungsmittel gewählt wird.

5. Verfahren nach Anspruch 1, dadurch gekennzeichnet, daß $R_3$ Alkenyl, Alkyl oder mit einer oder mehreren Carboxy-, Amido-, Cyano- oder Alkyloxycarbonylgruppen substituiertes Alkyl ist.

6. Verfahren nach Anspruch 1, dadurch gekennzeichnet, daß die Oximverbindung mit einem Alkalihydroxyid zu dem entsprechenden Alkalisalz der Oximverbindung umgesetzt wird.

7. Verfahren nach Anspruch 1, dadurch gekennzeichnet, daß es bei einer Temperatur zwischen 20°C und 150°C durchgeführt wird.

8. Verfahren nach Anspruch 1, dadurch gekennzeichnet, daß $R_1$ und $R_2$ gleich oder verschieden sind und Wasserstoff, Alkyl, Phenyl oder Phenylalkyl sind.

9. Verfahren nach Anspruch 1, dadurch gekennzeichnet, daß man das O-substituierte Oxim der Formel

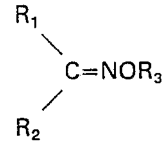

unter Bildung eines O-substituierten Hydroxylamins der Formel

$$H_2N—OR_3$$

hydrolysiert.

10. Verfahren nach Anspruch 1, dadurch gekennzeichnet, daß man das Oxim mit Dimethylsulfat unter Bildung eines Sulfatsalzes als Zwischenverbindung umsetzt, wonach man die Zwischenverbindung unter Bildung des entsprechenden N,O-substituierten Hydroxylamins und eines Aldehyds oder Ketons hydrolysiert.

**Revendications**

1. Procédé pour la préparation d'oximes O-substituées de formule:

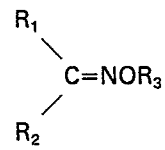

qui comprend les stades consistant à:

(a) faire réagir une oxime de formule:

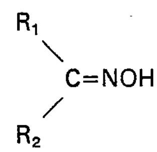

avec un hydroxyde de métal alcalin ou de métal alcalino-terreux dans un excès de ladite oxime pour former un mélange du sel de métal alcalin ou de métal alcalino-terreux de ladite oxime, d'eau et dudit excès de ladite oxime;

(b) soumettre le mélange réactionnel du stade (a) à une distillation azéotropique pour éliminer la totalité ou une partie de ladite eau dudit mélange; et

(c) faire réagir ledit sel de métal alcalin ou de métal alcalino-terreux de ladite oxime avec un composé de formule:

$$R_3X$$

dans laquelle:

X est un halogène;

$R_1$ et $R_2$ sont semblables ou différents et sont un hydrogène ou un arylthio, un arylsulfonyle, un arylsulfinyle, un alkylsulfinyle, un alkylsulfonyle,

un alkyle, un alcynyle, un cycloalkyle, un alcényle, un alcoxy, un alcoxyalkyle, un alkylthio, un aryle ou un aralkyle, substitués ou non substitués, ou $R_1$ et $R_2$ ensemble peuvent former une chaîne alkylène ou alcénylène substituée ou non substituée achevant un groupe cycloalkyle ou cycloalcényle contenant de 3 à environ 7 atomes de carbone dans la structure cyclique, où les substituants admissibles sont un ou plusieurs groupes alkyles, alcoxy, alkylthio, amido, carboxy, fluoro, nitro, cyano, alcoxycarbonyles, perfluoroalkyles, arylthio, phénoxy, alkylsulfinyles, alkylsulfonyles, arylsulfinyles, alkylaminocarbonyles, dialkylaminocarbonyles, alkylthiocarbonyles ou arylsulfonyles; et

$R_3$ est un fragment organique ayant un atome de carbone primaire, secondaire ou tertiaire lié à l'halogène dudit halogénure organique, dans lequel lesdits atomes de carbone dudit groupe $R_3$ peuvent être facultativement substitués par un ou plusieurs substituants qui sont inertes dans le procédé.

2. Procédé selon la revendication 1, dans lequel ledit mélange réactionnel soumis à une distillation azéotropique dans le stade (b) comprend un ou plusieurs solvants organiques inertes non polaires qui forment des mélanges azéotropiques lorsqu'ils sont distillés avec l'eau et ladite oxime.

3. Procédé selon la revendication 2, dans lequel ladite réaction du stade (a) est effectuée en présence d'un ou plusieurs solvants organiques inertes non polaires qui forment des mélanges azéotropiques lorsqu'ils sont distillés avec l'eau et ladite oxime.

4. Procédé selon la revendication 3, dans lequel ledit solvant est choisi dans le groupe constitué par les solvants hydrocarbonés aliphatiques et cycloaliphatiques, les solvants aromatiques et les solvants halogénohydrocarbonés.

5. Procédé selon la revendication 1, dans lequel $R_3$ est un alcényle, un alkyle ou un alkyle substitué par un ou plusieurs groupes carboxy, amido, cyano ou alcoxycarbonyles.

6. Procédé selon la revendication 1, dans lequel ladite oxime est mise à réagir avec un hydroxyde de métal alcalin pour former le sel de métal alcalin correspondant de ladite oxime.

7. Procédé selon la revendication 1, ledit procédé étant réalisé à une température entre 20°C et 150°C.

8. Procédé selon la revendication 1, dans lequel $R_1$ et $R_2$ sont semblables ou différents et sont un hydrogène, un alkyle, un phényle ou un phénylalkyle.

9. Procédé selon la revendication 1, qui comprend de plus l'hydrolyse de ladite oxime O-substituée de formule:

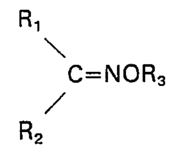

,

pour former une hydroxylamine O-substituée de formule:

$$H_2N—OR_3$$

10. Procédé selon la revendication 1, dans lequel ladite oxime est mise à réagir avec du sulfate de diméthyle pour produire un sel intermédiaire de type sulfate, lequel sel intermédiaire de type sulfate est ensuite hydrolysé pour produire l'hydroxylamine N,O-substituée correspondante et un aldéhyde ou une cétone.